# EUROPEAN PATENT SPECIFICATION

(11) **EP 2 654 663 B1**
(45) Date of publication and mention of the grant of the patent: **27.03.2019**
(21) Application number: 10805560.9
(22) Date of filing: 23.12.2010
(51) Int. Cl.: A61K 8/19, A61K 8/27, A61K 8/73, A61K 8/04, A61Q 11/00

(54) **FLUID ORAL CARE COMPOSITIONS**
FLÜSSIGE MUNDPFLEGEZUSAMMENSETZUNGEN
COMPOSITION FLUIDE DE SOINS BUCCAUX

(43) Date of publication of application: 30.10.2013
(73) Proprietor: Colgate-Palmolive Company, New York, NY 10022 (US)
(72) Inventor: NESTA, Jason, Cedar Knolls New Jersey 07927 (US); MARTINETTI, Melissa A., Bridgewater New Jersey 08807 (US); SZEWCZYK, Gregory, Flemington New Jersey 08822 (US); PIMENTA, Paloma, Staten Island New York 10306 (US)
(74) Representative: Wibbelmann, Jobst
(86) International application number: PCT/US2010/061956
(87) International publication number: WO 2012/087324

(56) References cited:
- EP-A2- 2 371 347
- DE-A1-102005 010 468
- JP-A- 8 003 074
- US-A1- 2008 145 505
- US-A1- 2010 189 663
- Dr. Hans M. Wyss, Ryan J. Larsen, Prof. David A. Weitz: "Oscillatory Rheology- Measuring the Viscoelastic Behaviour of Soft Materials", G.I.T. Laboratory Journal, vol. 3-4 2007, pages 68-70, XP002662839, Retrieved from the Internet: URL:http://www.mate.tue.nl/~wyss/files/Wys s_GIT_Lab_J_2007.pdf [retrieved on 2011-10-27]
- Galen W. Radebaugh, Anthony P. Simonelli: "Phenomenological viscoelasticity of a heterogeneous pharmaceutical semisolid", Journal of Pharmaceutical Sciences, vol. 72, no. 4 April 1983 (1983-04), pages 415-422, XP002662840, DOI: 10.1002/jps.2600720423 Retrieved from the Internet: URL:http://onlinelibrary.wiley.com/doi/10. 1002/jps.2600720423/pdf [retrieved on 2011-11-02]
- DATABASE CA [Online] CHEMICAL ABSTRACTS SERVICE, COLUMBUS, OHIO, US; 18 March 1997 (1997-03-18), EBINE, YOSHICHIKA ET AL: "Mouthwashes containing gellan gum", XP002662841, retrieved from STN Database accession no. 126:162020 & JP 8 337518 A (LION CORP, JAPAN) 24 December 1996 (1996-12-24)
- DATABASE CA [Online] CHEMICAL ABSTRACTS SERVICE, COLUMBUS, OHIO, US; 25 July 2002 (2002-07-25), KOJIMA, NOBUO: "Dentifrices containing gel-forming substances", XP002662842, retrieved from STN Database accession no. 137:67944 & JP 2002 193776 A (LION CORP., JAPAN) 10 July 2002 (2002-07-10)
- DATABASE CA [Online] CHEMICAL ABSTRACTS SERVICE, COLUMBUS, OHIO, US; 18 November 2004 (2004-11-18), YASUDA, NAOMI: "Mouthwash compositions containing disintegrating gels", XP002662843, retrieved from STN Database accession no. 141:370272 & JP 2004 300119 A (SUNSTAR, INC., JAPAN) 28 October 2004 (2004-10-28)

## Description

### BACKGROUND

The addition of zinc to oral care formulations may provide benefits such as: chelation of volatile sulfur compounds (VSC) which cause bad breath, inhibition of calculus formation, and anti-plaque/anti-gingivitis activity. However, due to the insoluble nature of zinc oxide, it is difficult to incorporate into a mouthwash in a way which is appealing to consumers.

While films are attractive additions to toothpastes and gels, they too precipitate and collect at the bottom of containers when incorporated into an oral rinse or mouthwash. Additionally, the presence of specks and glitter like particles in aqueous compositions has aesthetic appeal to some consumers. However, the insoluble nature of such decorative additives in oral rinse or mouthwash compositions requires consumers to resuspend the particles settled at the bottom of the container by shaking the composition.

There is a need for oral rinse and/or mouthwash compositions which can maintain insoluble materials and colloidal particles in suspension.

US-A-2010/189663 discloses mouth rinse (i.e., mouthwash) compositions that are intended to permit delivery of silica or silicate materials to the surface of teeth through common mouth rinsing procedures. US-A- 2008/145505 relates to gellan gum compositions having a 0.1% curdmeter gel strength of at least about 117 g/cm². JP-A-08-337518 describes a mouthwash composition comprising an abrasive in an amount in the range 2-30wt.% and having a viscosity of 1-1800cp at 25°C, wherein gellan gum is preferably added in an amount of 0.005-1wt.%. JP-A-2002-193776 discloses a cleaning agent for the oral cavity which is characterized by containing a jelly-like substance having ≥1mm average particle diameter. JP-A-08-003074 provides a liquid composition containing gellan gum and a nonionic surfactant such as a sucrose fatty acid ester, a polyoxyethylenesorbitan fatty acid ester, a polyoxyethylene-polyoxypropylene copolymer or a polyexyethylene-hardened castor oil. JP-A-2004-300119 describes a disintegrative gel comprising at least one first gelling agent selected from agar, carrageenan, furcellaran, alginic acid, its salt, gellan gum, and pectin, and at least one second gelling agent selected from glucomannan, native gellan gum, tara gum, locust bean gum, tamarind, celluloses and the like. DE-A-102005010468 discloses the use of shimmering food pigments comprising potassium aluminium silicate (E555), iron oxide (E172) and/or titanium dioxide (E171) in conjunction with softened water, sugar, gellan gum and trisodium citrate to form pearlescent gel beads or disks.

### SUMMARY

Structured oral rinse and mouthwash compositions which can maintain insoluble materials and colloidal particles in suspension are provided herein, thereby allowing for the delivery of insoluble active ingredients from a mouthwash without the inconvenience of agitating the mouthwash prior to use.

The present invention provides a fluid oral care composition comprising: from 0.05% to 0.3%, by weight, gellan gum; a sodium salt, wherein the sodium salt is present at a concentration of at least 0.5% by weight; xanthan gum; and an orally acceptable aqueous carrier; wherein the composition has a G'/G" ratio of greater than or equal to 1, and wherein the weight ratio of xanthan gum to gellan gum is from 3:7 to 1:1;the composition further comprising a suspended solid or semi-solid particle, wherein the solid or semi-solid particle is zinc oxide.

### DETAILED DESCRIPTION

As used throughout, ranges are used as shorthand for describing each and every value that is within the range. Any value within the range can be selected as the terminus of the range.

In the event of a conflict in a definition in the present disclosure and that of a cited reference, the present disclosure controls.

As used herein, the term "fluid composition" refers to a composition having the ability to take on the shape of its container, e.g. a mouthwash or an oral rinse.

As used herein, the term "aqueous" refers to a free water content of at least about 40%, by weight.

As used herein, the term "structuring agent" refers to a substance which is able to form by itself, or in combination with another substance, a structured network with a G / G" ratio 1.

In some embodiments, the composition comprises from 0.075 to 0.25%, by weight, gellan gum. In some embodiments, the composition comprises from 0.1 to 0.2%, by weight, gellan gum.

In some embodiments, polymer systems containing gellan gum are capable of building yield stress at much lower viscosities than most other hydrocolloids; allowing the system to suspend materials of dissimilar density while maintaining fluidity. In some embodiments, the combination of gellan gum, xanthan gum, and a sodium salt produces fluid compositions which can suspend insoluble materials and materials of dissimilar density.

The composition has a G'/G" ratio of greater than or equal to 1. In other embodiments, the composition has a G'/G" ratio of from 1 to 10. In some embodiments, the composition has a G' / G" ratio of from 2 to 9. In some embodiments, the composition has a G'/G" ratio of from 3 to 8. In some embodiments, the composition has a G'/G" ratio of from 4 to 7. In some embodiments, the composition has a G'/G" ratio of from 5 to 6. In further embodiments, the composition has a G'/G" ratio of 1.5. Still other embodiments provide compositions wherein the G'/G" ratio is 2. In some embodiments, the composition has a G'/G" ratio of 2.5. Yet other embodiments provide compositions wherein the G'/G" ratio is 3. While other embodiments provide compositions wherein the G'/G" ratio is 3.5. In some embodiments, the composition has a G'/G" ratio of 4. In some embodiments, the composition has a G'/G" ratio of 4.5. In some embodiments, the composition has a G'/G" ratio of 5. In some embodiments, the composition has a G'/G" ratio of 5.5. In some embodiments, the composition has a G'/G" ratio of 6. In some embodiments, the composition has a G'/G" ratio of 6.5. In some embodiments, the composition has a G'/G" ratio of 7. In some embodiments, the composition has a G'/G" ratio of 7.5. In some embodiments, the composition has a G'/G" ratio of 8. In some embodiments, the composition has a G'/G" ratio of 8.5. In some embodiments, the composition has a G'/G" ratio of 9. In some embodiments, the composition has a G'/G" ratio of 9.5.

Described are methods of treating or preventing a disease or condition of the oral cavity comprising contacting an oral cavity surface of a subject in need thereof, with any one of the compositions described herein. The disease or condition of the oral cavity may be xerostomia.

In some embodiments, the composition further comprises a humectant. In some embodiments, the humectant is selected from: glycerin; sorbitol; ethanol; propylene glycol; and a combination of two or more thereof. In some embodiments, the total humectant concentration is from 1 to 60%, by weight. Reference herein to sorbitol refers to the material typically available commercially in a 70% aqueous solution.

Other humectants such as polyol and sugar alcohol solutions may be present in amount of from about 1 to about 25% each, by weight. Sorbitol and/or another sugar alcohol are generally present, typically from about 1 to about 25%, by weight. In some embodiments, sorbitol is present at a concentration of from about 5 to about 15%, by weight. In other embodiments, sorbitol is present at a concentration of about 10%, by weight. Reference to sorbitol herein refers to the material typically available commercially as a 70% aqueous solution.

In some embodiments, glycerin and/or a similar polyol are present at a concentration of from about 1 to about 25% each, by weight. In some embodiments, glycerin is present at a concentration of from about 5 to about 15%, by weight. Some embodiments provide compositions wherein glycerin is present at a concentration of about 7.5%, by weight.

Another solvent, the diol propylene glycol, may be present. When present, propylene glycol is typically present at a concentration of from about 0.1 to about 50%, by weight. In some embodiments propylene glycol is present at a concentration of from about 5 to about 15%, by weight. Some embodiments have propylene glycol present at a concentration of about 7%, by weight. Other examples of humectant polyols include, but are not limited to: ethylene glycol; polyols, e.g. dipropylene glycol and hexylene glycol; cellosolves such as methyl cellosolve and ethyl cellosolve; vegetable oils and waxes containing at least about 12 carbons in a straight chain such as olive oil, castor oil and petrolatum; and esters such as amyl acetate, ethyl acetate and benzyl benzoate.

In some embodiments, the orally acceptable aqueous carrier comprises from about 40 to about 97%, by weight, free water. In some embodiments, the orally acceptable carrier comprises greater than about 40%, by weight, free water. In some embodiments, the orally acceptable carrier comprises greater than about 45%, by weight, free water. In some embodiments, the orally acceptable carrier comprises greater than 50%, by weight, free water. In some embodiments, the orally acceptable carrier comprises greater than about 55%, by weight, free water. In further embodiments, the orally acceptable aqueous carrier comprises greater than 60%, by weight, free water. In some embodiments, the orally acceptable carrier comprises greater than about 65%, by weight, free water. In some embodiments, the orally acceptable carrier comprises greater than about 70%, by weight, free water. In some embodiments, the orally acceptable carrier comprises about 70%, by weight, free water. In some embodiments, the orally acceptable carrier comprises about 71%, by weight, free water. In some embodiments, the orally acceptable carrier comprises about 72%, by weight, free water. In some embodiments, the orally acceptable carrier comprises about 73%, by weight, free water. In some embodiments, the orally acceptable carrier comprises about 74%, by weight, free water. In some embodiments, the orally acceptable carrier comprises about 75%, by weight, free water. In some embodiments, the orally acceptable carrier comprises greater than about 75%, by weight, free water. In some embodiments, the orally acceptable carrier comprises greater than about 80%, by weight, free water. In some embodiments, the orally acceptable carrier comprises greater than about 85%, by weight, free water. In some embodiments, the orally acceptable carrier comprises greater than about 90%, by weight, free water. In some embodiments, the orally acceptable carrier comprises greater than about 90%, by weight, free water.

In some embodiments, the water to humectant ratio is from 20:1 to 1:5. In some embodiments, the water to humectant ratio is from 10:1 to 1:3. In some embodiments, the water to humectant ratio is from 4:1 to 2:3.

The combination of polymers described herein imparts upon the product desirable structured attributes which allow the composition to maintain insoluble materials and materials of dissimilar density in suspension.

In some embodiments, the compositions described herein are adapted to suspend particulates, insoluble materials and colloidals which would otherwise precipitate shortly after being suspended in the liquid. The polymer combinations form a structure which - while having a network formed by the polymer mixture sufficient to maintain materials in suspension - remains in a liquid state. Non-limiting examples of suspended solid and semi-solid forms include flakes, specks, beads, and particulates.

Other optional additives may be included. Among such optional additives, included are those provided in order to change appearance or aesthetic appeal, and/or to preservative the final product, and/or for taste/cosmetic appeal and/or as therapeutic and prophylactic ingredients for oral health, prevention or treatment of a condition or disorder of hard or soft tissue of the oral cavity, or the prevention or treatment of a physiological disorder or condition.

In some embodiments, a preservative is present. In some embodiments a preservative is present in the amount of from about 0.0001 to about 1%, by weight. In some embodiments, the preservative is present at a concentration of about 0.5%, by weight. In some embodiments, the preservative is selected from parabens, potassium sorbate, benzyl alcohol, phenoxyethanol, polyaminopropryl biguanide, caprylic acid, sodium benzoate and cetylpyridinium chloride. In some embodiments, the preservative is sodium benzoate.

Colorants such as dyes may be food color additives presently certified under the Food Drug & Cosmetic Act for use in food and ingested drugs, including dyes such as FD&C Red No. 3 (sodium salt of tetraiodofluorescein), Food Red 17, disodium salt of 6-hydroxy-5-{(2-methoxy-5-methyl-4-sulphophenyl)azo}-2-naphthalenesulfonic acid, Food Yellow 13, sodium salt of a mixture of the mono and disulphonic acids of quinophtalone or 2-(2-quinolyl) indanedione, FD&C Yellow No. 5 (sodium salt of 4-p-sulfophenylazo-1-p-sul- fophenyl-5-hydroxypyrazole-3 carboxylic acid), FD&C Yellow No. 6 (sodium salt of p-sulfophenylazo-B-naphtol-6-monosulfonate), FD&C Green No. 3 (disodium salt of 4-{[4-(N-ethyl-p-sulfobenzylamino)-phenyl]-(4-hydroxy-2--sulfoniumphenyl)-methylene}-[1-(N-ethyl-N-p-sulfobenzyl)-.DELTA.-3,5-cycl-ohexadienimine], FD&C Blue No. 1 (disodium salt of dibenzyldiethyl-diamino- triphenylcarbinol trisulfonic acid anhydrite), FD&C Blue No. 2 (sodium salt of disulfonic acid of indigotin) and mixtures thereof in various proportions. Typically, colorants if included are present in very small quantities.

Flavoring agents are known, such as natural and artificial flavors. These flavorings may be chosen from synthetic flavor oils and flavoring aromatics, and/or oils, oleo resins and extracts derived from plants, leaves, flowers, fruits and so forth, and combinations thereof. Representative flavor oils include: spearmint oil, cinnamon oil, peppermint oil, clove oil, bay oil, thyme oil, cedar leaf oil, oil of nutmeg, oil of sage, and oil of bitter almonds. These flavoring agents can be used individually or in admixture. Commonly used flavors include mints such as peppermint, artificial vanilla, cinnamon derivatives, and various fruit flavors, whether employed individually or in admixture. Generally, any flavoring agent or food additive, such as those described in Chemicals Used in Food Processing, publication 1274 by the National Academy of Sciences, pages 63-258, may be used. Typically, flavoring agents, if included, are present at a concentration of from about 0.01 to about 1%, by weight. In some embodiments, the flavoring agent may be present at a concentration of about 0.2%, by weight.

Sweeteners include both natural and artificial sweeteners. Suitable sweeteners include water soluble sweetening agents such as monosaccharides, disaccharides and polysaccharides such as xylose, ribose, glucose (dextrose), mannose, galactose, fructose (levulose), sucrose (sugar), maltose, water soluble artificial sweeteners such as the soluble saccharin salts, i.e., sodium or calcium saccharin salts, cyclamate salts dipeptide based sweeteners, such a L-aspartic acid derived sweeteners, such as L-aspartyl-L-phenylalaine methyl ester (aspartame). In general, the effective amount of sweetener is utilized to provide the level of sweetness desired for a particular composition, will vary with the sweetener selected. This amount will normally be from about 0.001 to about 5%, by weight. In some embodiments, the sweetener is sodium saccharin and is present at a concentration of about 0.01%, by weight.

Whitening agents, material which is effective to effect whitening of a tooth surface to which it is applied, such as hydrogen peroxide and urea peroxide, high cleaning silica, preservatives, silicones, and chlorophyll compounds may be incorporated into the compositions of the present invention. In various embodiments, the compositions of this invention comprise a peroxide whitening agent, comprising a peroxide compound. A peroxide compound is an oxidizing compound comprising a bivalent oxygen-oxygen group. Peroxide compounds include peroxides and hydroperoxides, such as hydrogen peroxide, peroxides of alkali and alkaline earth metals, organic peroxy compounds, peroxy acids, pharmaceutically-acceptable salts thereof, and mixtures thereof. Peroxides of alkali and alkaline earth metals include lithium peroxide, potassium peroxide, sodium peroxide, magnesium peroxide, calcium peroxide, barium peroxide, and mixtures thereof. Organic peroxy compounds include carbamide peroxide (also known as urea hydrogen peroxide), glyceryl hydrogen peroxide, alkyl hydrogen peroxides, dialkyl peroxides, alkyl peroxy acids, peroxy esters, diacyl peroxides, benzoyl peroxide, and monoperoxyphthalate, and mixtures thereof. Peroxy acids and their salts include organic peroxy acids such as alkyl peroxy acids, and monoperoxyphthalate and mixtures thereof, as well as inorganic peroxy acid salts such as persulfate, dipersulfate, percarbonate, perphosphate, perborate and persilicate salts of alkali and alkaline earth metals such as lithium, potassium, sodium, magnesium, calcium and barium, and mixtures thereof. In various embodiments, the peroxide compound comprises hydrogen peroxide, urea peroxide, sodium percarbonate and mixtures thereof. In some embodiments, the peroxide compound comprises hydrogen peroxide. In some embodiments, the peroxide compound consists essentially of hydrogen peroxide. In some embodiments a non-peroxide whitening agent may be provided. Whitening agents among those useful herein include non-peroxy compounds, such as chlorine dioxide, chlorites and hypochlorites. Chlorites and hypochlorites include those of alkali and alkaline earth metals such as lithium, potassium, sodium, magnesium, calcium and barium. Non-peroxide whitening agents also include colorants, such as titanium dioxide and hydroxyapatite. One or more whitening agents are optionally present in a tooth-whitening effective total amount. In some embodiments the whitening agent is separated from the aqueous carrier. In some embodiments the whitening agent is separated from the aqueous carrier by encapsulation of the whitening agent.

Optionally, breath freshening agents may be provided. Any orally acceptable breath freshening agent can be used, including without limitation zinc salts such as zinc gluconate, zinc oxide, zinc citrate and zinc chlorite, alpha-ionone and mixtures thereof. One or more breath freshening agents are optionally present in a breath freshening effective total amount.

Optionally, the composition may include a tartar control (anticalculus) agent. Tartar control agents among those useful herein include phosphates and polyphosphates (for example pyrophosphates), polyaminopropanesulfonic acid (AMPS), polyolefin sulfonates, polyolefin phosphates, diphosphonates such as azacycloalkane-2,2-diphosphonates (e.g., azacycloheptane-2,2-diphosphonic acid), N-methyl azacyclopentane-2,3-diphosphonic acid, ethane-1-hydroxy-1,1-diphosphonic acid (EHDP) and ethane-1-amino-1,1-diphosphonate, phosphonoalkane carboxylic acids and salts of any of these agents, for example their alkali metal and ammonium salts. Useful inorganic phosphate and polyphosphate salts include monobasic, dibasic and tribasic sodium phosphates, sodium tripolyphosphate, tetrapolyphosphate, mono-, di-, tri- and tetrasodium pyrophosphates, sodium trimetaphosphate, sodium hexametaphosphate and mixtures thereof, wherein sodium can optionally be replaced by potassium or ammonium. Other useful anticalculus agents include polycarboxylate polymers and polyvinyl methyl ether/maleic anhydride (PVME/MA) copolymers, such as those available under the Gantrez™ brand from ISP, Wayne, N.J. In some embodiments, a phosphate is present at a concentration of from about 0.01 to about 10%, by weight. In some embodiments, a phosphate is present at a concentration of from about 1%, by weight.

Other optional additives include antimicrobial (e.g., antibacterial) agents. Any orally acceptable antimicrobial agent can be used, including triclosan (5-chloro-2-(2,4-dichlorophenoxy)phenol); zinc and stannous ion sources; quaternary ammonium compounds such as cetylpyridinium chloride (CPC); bisguanides such as chlorhexidine; and benzalkonium chloride. A further illustrative list of useful antibacterial agents is provided in U.S. Pat. No. 5,776,435 to Gaffar, et al.

Antioxidants are another class of optional additives. Any orally acceptable antioxidant can be used, including butylated hydroxyanisole (BHA), butylated hydroxytoluene (BHT), vitamin A, carotenoids, vitamin E, flavonoids, polyphenols, ascorbic acid, herbal antioxidants, chlorophyll, melatonin, and mixtures thereof.

Also optional, saliva stimulating agent, useful for example in amelioration of dry mouth may be included. Any orally acceptable saliva stimulating agent can be used, including without limitation food acids such as citric, lactic, malic, succinic, ascorbic, adipic, fumaric, and tartaric acids, and mixtures thereof. One or more saliva stimulating agents are optionally present in a saliva stimulating effective total amount.

Optionally, an antiplaque (e.g., plaque disrupting) agent may be included. Any orally acceptable antiplaque agent can be used, including without limitation stannous, copper, magnesium and strontium salts, dimethicone copolyols such as cetyl dimethicone copolyol, papain, glucoamylase, glucose oxidase, urea, calcium lactate, calcium glycerophosphate, strontium polyacrylates and mixtures thereof.

Optional desensitizing agents include potassium citrate, potassium chloride, potassium tartrate, potassium bicarbonate, potassium oxalate, potassium nitrate, strontium salts, and mixtures thereof. In some embodiments, a local or systemic analgesic such as aspirin, codeine, acetaminophen, sodium salicylate or triethanolamine salicylate can be used.

Optional additives also include nutrients and/or proteins. Suitable nutrients include vitamins, minerals, amino acids, and mixtures thereof. Vitamins include Vitamins C and D, thiamine, riboflavin, calcium pantothenate, niacin, folic acid, nicotinamide, pyridoxine, cyanocobalamin, para-aminobenzoic acid, bioflavonoids, pantheon, retinyl palmitate, tocopherol acetate, and mixtures thereof. Nutritional supplements include amino acids (such as L-tryptophane, L-lysine, methionine, threonine, levocamitine and L-carnitine), lipotropics (such as choline, inositol, betaine, and linoleic acid), fish oil (including components thereof such as omega-3 (N-3) polyunsaturated fatty acids, eicosapentaenoic acid and docosahexaenoic acid), coenzyme Q10, and mixtures thereof. Herbs such as *Chamomilla recutita, Mentha piperita, Salvia officinalis, and Commiphora myrrha* may optionally be included. Suitable proteins include milk proteins and enzymes such as peroxide-producing enzymes, amylase, plaque-disrupting agents such as papain, glucoamylase and glucose oxidase.

Compositions of the present invention may be made using a process in which the gellan and xanthan gums are first hydrated and mixed with a sodium salt at an elevated temperature and then mixed with the remaining ingredients after cooling. Water is initially heated to a temperature sufficient to hydrate said xanthan gum and said gellan gum. In some embodiments, the temperature of the water used for hydrating the gums is from about 65.6°C (about 150°F) to about 93.3°C (about 200°F). In some embodiments, the temperature of the water used for hydrating the gums is between 71.1°C and 82.2°C (160°F and 180°F). In some embodiments, the temperature of the water used for hydrating the gums is about 82.2°C (about 180°F).

In some embodiments, the xanthan gum and gellan gum are then added to the heated water and maintained for a time and at a temperature sufficient to hydrate the xanthan gum and the gellan gum. In some embodiments, the mixture is maintained at a temperature of from about 65.6°C to about 93.3°C (about 150°F to about 200°F). In some embodiments, the mixture is maintained at a temperature of from about 71.1°C (about 160°F) to 82.2°C (180°F). In some embodiments, the mixture is maintained at a temperature of about 82.2°C (180°F).

In some embodiments, the mixture is maintained at a temperature of from about 65.6°C to about 93.3°C (about 150°F to about 200°F), for from about 3 minutes to about 30 minutes. In some embodiments, the mixture is maintained at a temperature of from about 65.6°C to about 93.3°C (about 150°F to about 200°F), for from about 5 minutes to about 15 minutes. In some embodiments, the mixture is maintained at a temperature of from about 65.6°C to about 93.3°C (about 150°F to about 200°F), for about 15 minutes.

In some embodiments, the mixture is maintained at a temperature of from about 71.1°C to about 82.2°C (about 160°F to about 180°F), for from about 3 minutes to about 30 minutes. In some embodiments, the mixture is maintained at a temperature of from about 71.1°C to about 82.2°C (about 160°F to about 180°F), for from about 5 minutes to about 15 minutes. In some embodiments, the mixture is maintained at a temperature of from about 71.1°C to about 82.2°C (about 160°F to about 180°F), for about 15 minutes.

In some embodiments, the mixture is maintained at a temperature of about 82.2°C (about 180°F), for from about 3 minutes to about 30 minutes. In some embodiments, the mixture is maintained at a temperature of about 82.2°C (about 180°F), for from about 5 minutes to about 15 minutes. In some embodiments, the mixture is maintained at a temperature of about 82.2°C (about 180°F), for about 15 minutes.

The mixture may be mixed for all or part of the time it is maintained at the temperature sufficient to hydrate the xanthan gum and the gellan gum. In some embodiments, the mixture is mixed for at least about 3 minutes and no longer than 30 minutes. In some embodiments, the mixture is mixed for 5 to 15 minutes. In some embodiments, the mixture is mixed for about 15 minutes. Following this step the mixture comprises hydrated gellan and xanthan gums.

Sodium salt is then added to the mixture. In some embodiments, from about 0.5 to about 1%, by weight, of sodium salt is added.

The mixture of hydrated gums and sodium salt is allowed to cool to a temperature of about 51.7°C (about 125°F) or less. In some embodiments, the mixture of hydrated gums and sodium salt is cooled to a temperature from about 23.9°C to about 43.3°C (75°F to about 110°F). In some embodiments, the mixture of hydrated gums and sodium salt is cooled to about 23.9°C (about 75°F). In some embodiments, the mixture of hydrated gums and sodium salt is mixed for all or part of the time it is cooling. In some embodiments, mixing is discontinued during the entire cooling.

Once the mixture has cooled, the remaining ingredients are added to the cooled mixture to produce a combined composition. In some embodiments, the remaining ingredients include a material of dissimilar density. In some embodiments, the material of dissimilar density is a solid or semi-solid particle.

The combined composition is mixed to incorporate the added ingredients, preferably essentially to a homogenous degree. In some embodiments, the combined composition is mixed for from about 5 to about 60 minutes. In some embodiments, the combined composition is mixed for from about 10 to about 30 minutes. In some embodiments, the combined composition is mixed for about 15 minutes. In some embodiments, the combined composition is mixed for about 15 minutes. The combined composition may be preferably mixed at a low or medium speed to avoid foaming. In some embodiments, the combined composition is mixed at a medium speed to avoid foaming.

The following examples (outside the scope of the claims) serve to illustrate certain aspects of the present invention.

### EXAMPLES

### Example 1

Table 1 (below) describes the formulations of exemplary compositions.

**Table 1**

| Ingredient | % (w/w) | Range |
|---|---|---|
| Vegetable glycerin | 7.5 | 0-40% |
| Sorbitol (70% solution) | 5.5 | 0-40% |
| Propylene glycol | 7 | 0-40% |
| Polysorbate 20 | 1 | 0.1-3% |
| Disodium phosphate | 0.5 | 0.25-2% |
| Flavor | 0.12 | 0.05-0.5% |
| Sodium benzoate | 0.5 | 0.0001-0.5% |
| Cetylpyridinium chloride | 0.05 | 0.045-0.1% |
| Gellan gum | 0.07 | 0.05-0.3% |
| Sodium fluoride | 0.05 | 0-0.05% |
| Sodium saccharin | 0.02 | 0.001-0.05% |
| Xanthan gum | 0.03 | 0-0.3% |
| Simethicone emulsion | 0.001 | 0.001% |
| Colorant | 0 | 0-0.1% |
| 95% Ethanol | 0 | 0-12 |
| Water | *77.7* | q.s. |

### Example 2

A series of 21 formulations were developed to identify the formula parameters necessary to suspend a particle of dissimilar density in an oral rinse solution. The effects of manipulating the levels of gellan gum, xanthan gum, disodium phosphate, glycerin and water, on the structural properties of the finished product were identified.

A laboratory rheometer was used to analyze the rheological properties of each product made.

All of the experiments conducted as part of this work were performed in a stress controlled AR2000 rheometer (TA Instruments), using a cuette/cylinder geometry. A peltier was used to control the temperature and a solvent trap was used to prevent sample evaporation. All experiments were performed at 25±0.1°C unless otherwise indicated.

In a strain sweep experiment, the amplitude of the applied strain varies in the range 0.1% < y < 100% while the frequency of oscillations is kept constant. The viscoelastic response of the material to the applied oscillatory strain is measured in terms of G' and G", the viscous and loss moduli, and valuable information is obtained this way. In general, G' represents energy storage within the viscoelastic structure and G" represents dissipation of this energy through flow. The linear viscoelastic region (LVR) is determined by the region of the strain sweep in which G' and G" remain constant with respect to the applied strain and the ratio of elastic to viscous contribution (G'/G") can be calculated based on the G' and G" values within the LVR. This ratio provides a good indication of how structured the mouthwash base is, with a higher G'/G" ratio indicating that a more robust structure is present within the system. The yield stress value is also determined from a strain sweep experiment, by plotting the elastic stress (G' x Strain) vs. Strain.

With this information at hand one can determine whether a certain viscoelastic material exhibits more solid-like or more fluid-like properties, and in this particular case the data can be utilized effectively to determine whether various aesthetics and solid materials can be successfully suspended within the mouthwash.

Just as structural properties are characterized by oscillatory experiments, flow properties of different materials can be characterized through steady state shearing. In a steady state flow experiment a range of strains (shear rates) is applied to the sample and the viscosity, and/or resulting shear stress are plotted as a function of the applied shear rate. Flow curves were obtained here in the shear rate range of 0.1 to 100 sec⁻¹. The viscosity at a single, arbitrary shear rate was then used to characterize a particular sample and compare it to other samples.

Tables 2 and 3 (below) describe the formula parameters of exemplary compositions that were evaluated in the strain sweep experiments described in this Example.

**Table 2**

| **Ingredient** | **% (w/w)** |
|---|---|
| Vegetable glycerin | 7.5-25 |
| Sorbitol (70% solution) | 5.5 |
| Propylene glycol | 7 |
| Polysorbate 20 | 1 |
| Disodium phosphate | 0.5-1 |
| Flavor | 0.12 |
| Sodium benzoate | 0.5 |
| Cetylpyridinium chloride | 0.05 |
| Gellan gum | 0.025-0.084 |
| Sodium fluoride | 0.05 |
| Sodium saccharin | 0.02 |
| Xanthan gum | 0.018-0.070 |
| Simethicone emulsion | 0.001 |
| Water | q.s. |

**Table 3**

| **Formula** | **Gellan % (w/w)** | **Xanthan % (w/w)** | **Disodium Phosphate % (w/w)** | **Glycerin % (w/w)** |
|---|---|---|---|---|
| 1 | 0.05 | 0.04 | 1 | 7.5 |
| 2 | 0.042 | 0.018 | 1 | 7.5 |
| 3 | 0.025 | 0.035 | 1 | 7.5 |
| 4 | 0.025 | 0.035 | 0.5 | 7.5 |
| 5 | 0.042 | 0.018 | 0.5 | 7.5 |
| 6 | 0.063 | 0.027 | 0.75 | 7.5 |
| 7 | 0.05 | 0.07 | 0.5 | 7.5 |
| 8 | 0.034 | 0.026 | 0.75 | 7.5 |
| 9 | 0.05 | 0.07 | 1 | 7.5 |
| 10 | 0.067 | 0.053 | 0.75 | 7.5 |
| 11 | 0.05 | 0.04 | 0.75 | 7.5 |
| 12 | 0.038 | 0.052 | 0.75 | 7.5 |
| 13 | 0.084 | 0.036 | 0.5 | 7.5 |
| 14 | 0.05 | 0.04 | 0.5 | 7.5 |
| 15 | 0.084 | 0.036 | 1 | 7.5 |
| 16 | 0.05 | 0.04 | 0.75 | 7.5 |
| 17 | 0.05 | 0.04 | 0.75 | 7.5 |
| 18 | 0.05 | 0.04 | 0.75 | 11.9 |
| 19 | 0.05 | 0.04 | 0.75 | 25 |
| 20 | 0.05 | 0.04 | 0.75 | 20.6 |
| 21 | 0.05 | 0.04 | 0.75 | 16.3 |

Table 4 (below) describes the data generated from the strain sweep experiments conducted on exemplary compositions.

**Table 4**

| Formula | G' (dyne/cm²) | G" (dyne/cm²) | G'/G" | Yield Stress (dyne/cm²) |
|---|---|---|---|---|
| 1 | 25.3 | 9.4 | 2.7 | 3.3 |
| 2 | 13.9 | 4.5 | 3.1 | 2.8 |
| 3 | 1.8 | 1.9 | 1 | 1.1 |
| 4 | 2.5 | 2.4 | 1 | 1 |
| 5 | 11.9 | 4.8 | 2.5 | 1.3 |
| 6 | 36.8 | 11.9 | 3.1 | 2.3 |
| 7 | 49.1 | 17.4 | 2.8 | 2.5 |
| 8 | 7.2 | 3.9 | 1.9 | 1.9 |
| 9 | 23.5 | 12.6 | 1.9 | 2.3 |
| 10 | 90.4 | 27.4 | 3.3 | 3.4 |
| 11 | 34.1 | 12 | 2.8 | 3.8 |
| 12 | 16.3 | 7.9 | 2.1 | 4.1 |
| 13 | 198.3 | 39.3 | 5 | 4.5 |
| 14 | 29.2 | 13.6 | 2.2 | 3.9 |
| 15 | 198.6 | 50.2 | 4 | 5.3 |
| 16 | 14.4 | 6.5 | 2.2 | 2.3 |
| 17 | 16.1 | 9.1 | 1.8 | 1.6 |
| 18 | 26.1 | 12.8 | 3.3 | 4.6 |
| 19 | 38 | 7.5 | 3.2 | 6.2 |
| 20 | 36.9 | 7.8 | 2.9 | 5.8 |
| 21 | 28.2 | 7.9 | 3.6 | 4.1 |

The data described in this Example demonstrates that gellan gum concentration is the primary factor affecting G'/G", yield stress, and viscosity. However, xanthan gum also has significant influence on these properties. As the gum proportion shifts towards xanthan, both G'/G" and yield stress decrease,
suggesting depreciation of the products suspension capabilities. The viscosity changes in a very similar manner to the yield stress, indicating that an increase in viscosity must be accepted when trying to suspend denser particles.

### Example 3

Table 5 (below) provides the formulation of an exemplary composition (Composition A).

**Table 5**

| Ingredient | % w/w |
|---|---|
| Water | 71.96 |
| Vegetable Glvcerin | 7.5 |
| Ethyl Alcohol | 6 |
| Sorbitol (70% Solution) | 5.5 |
| Propylene Glycol | 5 |
| Polysorbate 20 | 2 |
| Anhydrous Disodium Phosphate | 0.75 |
| Flavor | 0.4 |
| Sodium Benzoate | 0.25 |
| Cetylpyridinium Chloride | 0.05 |
| Gellan Gum | 0.05 |
| Sodium Fluoride | 0.05 |
| Sodium Saccharin | 0.05 |
| Xanthan Gum | - |
| Simethicone Emulsion | 0.001 |

Table 6 (below) describes a comparison of the rheology parameters of Composition A and a commercially available product containing a polymer system. The data described in Table 6, illustrates that the polymer systems of the present invention provide a structured system capable of suspending a material with dissimilar density, while the Comparative Example would not.

**Table 6**

| Composition | G' (dyn/cm2) | G" (dyn/cm2) | Structural Parameter (G'/G") |
|---|---|---|---|
| A | 63.6 | 11.3 | 5.7 |
| Comp Ex 1 | 0.01 | 0.41 | 0.02 |

Comparative Example 1 (Comp Ex 1) contains the following ingredients: water, glycerin, sorbitol, poloxamer 338, PEG-60 hydrogenated castor oil, carboxymethylcellulose, cetylpyridinium chloride, copovidone, propylparaben, sodium benzoate, sodium phosphate, sodium saccharin, xanthan gum, and FD&C blue no. 1.

## Claims

1. A fluid oral care composition comprising:
from 0.05% to 0.3%, by weight, gellan gum;
a sodium salt, wherein the sodium salt is present at a concentration of at least 0.5% by weight;
xanthan gum; and
an orally acceptable aqueous carrier;
wherein the composition has a G'/G" ratio of greater than or equal to 1, and wherein the weight ratio of xanthan gum to gellan gum is from 3:7 to 1:1;
the composition further comprising a suspended solid or semi-solid particle, wherein the solid or semi-solid particle is zinc oxide.

2. The composition of claim 1, wherein the total gum concentration is from 0.05 to 0.12% by weight.

3. The composition of claim 1 or claim 2, wherein the composition has a G'/G" ratio of from 1 to 10.

4. The composition of claim 3, wherein the composition has a G'/G" ratio of from 5 to 6.

5. The composition of claim 4, wherein the composition has a G'/G" ratio of 5.5.

6. The composition of any preceding claim, further comprising a humectant.

7. The composition of claim 6, wherein the humectant is selected from: glycerin; sorbitol; ethanol; propylene glycol; and a combination of two or more thereof.

8. The composition of any preceding claim, wherein the orally acceptable aqueous carrier has a free water concentration of greater than 50%, by weight.

9. The composition of any one of claims 6 to 8, wherein the water to humectant ratio is from 4:1 to 2:3.

## Patentansprüche

1. Fluide Mundpflegezusammensetzung umfassend:
von 0,05 bis 0,3 Gew.-%Gellangummi;
ein Natriumsalz, worin das Natriumsalz bei einer Konzentration von mindestens 0,5 Gew.-% vorliegt;
Xanthangummi; und
einen oral annehmbaren wässrigen Träger;
wobei die Zusammensetzung ein G'/G"-Verhältnis von größer als oder gleich 1 hat und worin das Gewichtsverhältnis Xanthangummi zu Gellangummi von 3:7 bis 1:1 ist;
wobei die Zusammensetzung weiterhin ein suspendiertes festes oder semi-festes Teilchen umfasst, wobei das feste oder semi-feste Teilchen Zinkoxid ist.

2. Zusammensetzung nach Anspruch 1, worin die gesamte Gummikonzentration von 0,05 bis 0,12 Gew.-% ist.

3. Zusammensetzung nach Anspruch 1 oder Anspruch 2, wobei die Zusammensetzung ein G'/G"-Verhältnis von 1 bis 10 hat.

4. Zusammensetzung nach Anspruch 3, worin die Zusammensetzung ein G'/G"-Verhältnis von 5 bis 6 hat.

5. Zusammensetzung nach Anspruch 4, worin die Zusammensetzung ein G'/G"-Verhältnis von 5,5 hat.

6. Zusammensetzung nach irgendeinem vorhergehenden Anspruch, weiterhin umfassend ein Befeuchtungsmittel.

7. Zusammensetzung nach Anspruch 6, worin das Befeuchtungsmittel ausgewählt ist aus: Glycerin, Sorbitol, Ethanol, Propylenglycol und einer Kombination aus zwei oder mehreren davon.

8. Zusammensetzung nach irgendeinem vorhergehenden Anspruch, wobei der oral annehmbare wässrige Träger eine freie Wasserkonzentration von größer als 50 Gew.-% hat.

9. Zusammensetzung nach irgendeinem der Ansprüche 6 bis 8, worin das Wasser zu Befeuchtungsmittel-Verhältnis von 4:1 bis 2:3 ist.

## Revendications

1. Composition de soin buccal fluide comprenant :
de 0,05 % à 0,3 %, en poids, de gomme gellane ;
un sel de sodium, dans laquelle le sel de sodium est présent à une concentration d'au moins 0,5 % en poids ;
de la gomme xanthane ; et
un support aqueux acceptable par voie orale ;
dans laquelle la composition présente un rapport G'/G" supérieur ou égal à 1, et dans laquelle le rapport pondéral de la gomme xanthane à la gomme gellane est compris entre 3:7 et 1:1 ;
la composition comprenant en outre une particule solide ou semi-solide en suspension, dans laquelle la particule solide ou semi-solide est de l'oxyde de zinc.

2. Composition selon la revendication 1, dans laquelle la concentration totale en gommes est comprise entre 0,05 et 0,12 % en poids.

3. Composition selon la revendication 1 ou la revendication 2, dans laquelle la composition a un rapport G'/G" compris entre 1 et 10.

4. Composition selon la revendication 3, dans laquelle la composition a un rapport G'/G" compris entre 5 et 6.

5. Composition selon la revendication 4, dans laquelle la composition a un rapport G'/G" de 5,5.

6. Composition selon l'une quelconque des revendications précédentes, comprenant en outre un humectant.

7. Composition selon la revendication 6, dans laquelle l'humectant est choisi parmi : la glycérine ; le sorbitol ; l'éthanol ; le propylène glycol ; et une combinaison de deux ou plus de ceux-ci.

8. Composition selon l'une quelconque des revendications précédentes, dans laquelle le support aqueux acceptable par voie orale a une concentration en eau libre supérieure à 50 %, en poids.

9. Composition selon l'une quelconque des revendications 6 à 8, dans laquelle le rapport eau sur humectant est compris entre 4:1 et 2:3.
